# EUROPEAN PATENT SPECIFICATION

(11) **EP 1 082 286 B1**
(45) Date of publication and mention of the grant of the patent: **23.02.2005**
(21) Application number: 99923195.4
(22) Date of filing: 18.05.1999
(51) Int. Cl.: C07C 51/43, C07C 63/24, C07C 63/26

(54) **METHOD FOR PREPARING PURIFIED TEREPHTHALIC ACID AND ISOPHTHALIC ACID FROM MIXED XYLENES**
VERFAHREN ZUR REINDARSTELLUNG VON TEREPHTHALSÄURE UND ISOPHTHALSÄURE AUS XYLOLGEMISCHEN
PROCEDE DE PREPARATION D'ACIDE TEREPHTALIQUE ET D'ACIDE ISOPHTALIQUE PURIFIES A PARTIR DE MELANGES DE XYLENES

(30) Priority: 29.05.1998 US 87141 P; 16.06.1998 US 97930
(43) Date of publication of application: 14.03.2001
(73) Proprietor: GTC Technology, Inc, Houston, Texas 77077 (US)
(72) Inventor: LEE, Fu-Ming, Katy, TX 77450 (US); LAMSHING, Wiston, Katy, TX 77450 (US); WYTCHERLEY, Randi, Wright, Belgrade, MT 59714 (US)
(74) Representative: Nash, David Allan
(86) International application number: PCT/US1999/010970
(87) International publication number: WO 1999/062857

(56) References cited:
- WO-A-96/40612
- WO-A-98/24749
- WO-A-99/23055
- GB-A- 818 211
- GB-A- 849 189
- US-A- 2 949 483

## Description

### INTRODUCTION

The present invention relates to the production of terephthalic and isophthalic acids and, more specifically, to a method for preparing purified terephthalic acid and isophthalic acid from mixed xylenes.

### BACKGROUND

Conventional terephthalic acid (TPA) manufacturing processes require relatively high p-xyiene purity (99.7+ %) in order to improve the quality of the product and reduce the costs of manufacturing. This is due to the fact that such prior art processes use hydrogenation as the main method for purifying the crude terephthalic acid produced in the oxidation section of said processes. Although the hydrogenation method is very selective to. eliminate the major impurity, 4-carboxybenzaldehyde (4-CBA) by converting it to p-toluic acid, the method only operates in the presence of a very small amount of 4-CBA (preferably less than 3,000 ppm). Also, the conventional TPA manufacturing processes are not capable of separating TPA from its isomers, such as isophthalic acid (IPA) and phthalic acid (PA).

Other background documents include GB 818 211 A, by Jock Usch *et*. *al*, which teaches a method for the manufacture of terephthalic acid wherein N:N-dimethyl formamide is used as a solvent to recrystallize terephthalic acid and to free it from impurities. The reference teaches that by dissolving crude terephthalic acid at the boiling temperature of the solvent followed by cooling, it is possible to obtain a pure form of terephthalic acid.

GB 849 189 A, by Bille *et al,* relates to a process wherein organic impurities are removed from terephthalic acid by recrystallization from organic solvents, specifically from an N-alkyl-pyrrolidine.

US 2,949,483, by Ham, relates to a method purifying aromatic dicarboxylic acids used in the manufacture of fiber- and filament-forming polyesters and copolyesters, by means of recrystallization from organic solvents.

### SUMMARY OF INVENTION

In contrast to the prior art TPA processes described above, the present invention provides a method and apparatus for preparing purified terephthalic acid and isophthalic acid from mixed xylenes. Importantly, it can purify the oxidation reactor effluent containing the mixture of terephthalic acid and isophthalic acid as well as other impurities such as minor amounts of 4-carboxybenzaldehyde (4-CBA), 3-carboxybenzaldehyde (3-CBA), and toluic acid isomers, to produce a purified terephthalic acid and purified isophthalic acid in an integrated process. These products are useful for the production of fibers, films, plastic bottles, and polyester resin structures, often reinforced by other materials such as glass fiber.

In accordance with the present invention there is provided a process for separating and purifying crude terephthalic acid (TPA) and isophthalic acid (IPA), from a liquid dispersion consisting of TPA, IPA and other impurities produced from the oxidation of mixed xylenes comprising:
(a) dissolving the crude TPA in a selective crystallization solvent at a temperature of from 50°C to 250°C to form a solution;
(b) crystallizing purified acid from said solution by reducing the temperature and/or pressure thereof, and separating said crystallized purified TPA from said solution;
(c) adding an anti-solvent to the said solution from which said crystallized purified TPA is separated to cause the precipitation of substantially all the TPA remaining in said solution;
(d) separating said precipitated TPA;
(e) evaporating the solvents from the solution to cause crystallization of IPA, and purifying and recovering purified IPA thereby.
In one embodiment, the process of the present invention includes the production of crude mixed acids (including terephthalic acid and isophthalic acid) by the oxidation of the mixed xylenes containing mainly p-xylene and smaller portions of m-xylene and other isomers. The oxidation step produces not only terephthalic acid and isophthalic acid, but also, by incomplete oxidation other impurities such as 4-CBA, 3-CBA, p-toluic acid, m-toluic acid and other trace amounts of acid and aldehyde isomers. The product resulting from the oxidation step is a liquid dispersion containing unreacted starting materials, solvents, if any have been used, the products of side reactions, particularly those just mentioned, and other materials which are not desired in the sought-for purified terephthalic acid and purified isophthalic acid.

The reactor effluent may be fed to a series of crystallizers which allow the solids to grow by evaporating the reaction solvent, preferably acetic acid, through pressure reductions. The slurry from the last crystallizer is filtered and washed. The filtered crystals are then dried to remove the solvent to a level ofless than 0.25% in the resulting crude mixed acid crystals. The mother liquor from the filtration is fed to the solvent dehydration unit to recover the solvent (acetic acid) from water for recycling to the oxidizer.

In further accordance with the invention, the crude mixed acids from the dryer of the oxidation section are re-dissolved in a selective crystallization solvent and then terephthalic acid (TPA) is crystallized out of the selective crystallization solvent for example in one or, preferably, two crystallization stages. Provision is made to separate out the crystallized and progressively purified TPA from the solvent (with or without cosolvents) of the invention. The filter cake of purified TPA ultimately obtained is washed and soaked with water to remove color and the final trace of the selective crystallization solvent from the TPA product.

In order to recover isophthalic acid (IPA) from the crystallizer mother liquor (after TPA solids are removed by filtration), an anti-solvent is added to cause the substantially complete precipitation of TPA from the mother liquor. The substantially TPA-free mother liquor is concentrated, by evaporating the selective crystallization solvent and the anti-solvent, from the mother liquor, and cooled to cause the crystallization of crude IPA. The crude IPA may then be further purified by recrystallizihg in another selective crystallization solvent.

The invention also contemplates steps to reclaim and recycle the solvents of the invention at each stage of crystallization and washing, and final soaking. Steps are also taken to closely control the delivery of any objectionable materials to the environment.

One important aspect of the present invention is the discovery of solvents which are effective to bring about the purification of TPA as well as IPA from a crude mixture containing TPA, up to 20% IPA, smaller quantities of 3-CBA, 4-CBA, m-toluic acid, p-toluic acid, and others, through crystallization and separation steps. These discoveries may be summarized as follows.

For TPA purification, the selective crystallization solvents useful in the practice of the present invention include those in which (a) the impurities (including IPA) desired to be separated from TPA are relatively more soluble in the solvent than is TPA at substantially every temperature within the desired range of temperatures at which the solvent containing TPA is to be handled, and (b) TPA is more soluble at an elevated temperature and less soluble at a lower or reduced temperature. It is to be understood that the term "selective crystallization solvent" is intended to include any solvents useful in the selective crystallization of TPA as described above.

For IPA purification, the anti-solvent which is to be added to the mother liquor (from TPA crystallization effluent) should cause substantially total precipitation (or crystallization) of TPA from the mother liquor and yet retain the major portion of IPA in the mother liquor. The substantially TPA-free mother liquor is concentrated by evaporation (or distillation) to crystallize crude IPA, which may then be separated by filtration and redissolved in a second selective crystallization to yield the purified IPA.

In accordance with the invention, the primary preferred selective crystallization solvent for purifying TPA is N-methyl pyrrolidone (NMP), for the several reasons discussed below, and for its superior performance. It is non-aqueous, thermally stable, non-toxic (environmentally safe), non-corrosive, and commercially available. TPA can be dissolved in NMP at elevated temperatures, and precipitated or crystallized from NMP at lower temperatures. The major impurities such as 4-CBA, 3-CBA, p-toluic acid, m-toluic acid, as well as IPA, have relatively higher solubility in NMP than TPA at all temperatures. Therefore, by lowering the temperature, only TPA tends to crystallize or precipitate from the solution to form purified TPA crystals.

Although NMP is the most preferred selective crystallization solvent, it is to be understood that, in accordance with the present invention, other preferred selective crystallization solvents for purification of crude TPA can be selected from various polar organic solvents including, but not intended to be limited to, N,N-dimethyl acetamide, N,N-dimethyl formamide, N-formyl piperidine, N-alkyl-2-pyrrolidone (such as N-ethyl pyrrolidone), N-mercaptoalkyl-2-pyrrolidone (such as N-mercaptoethyl-2-pyrrolidone), N-alkyl-2-thiopyrrolidone (such as N-methyl-2-thiopyrrolidone), N-hydroxyalkyl-2-pyrrolidone (such as N-hydroxyethyl-2-pyrrolidone), the morpholines (such as morpholine, and N-formyl morpholine), the carbitols, C₁ to C₁₂ alcohols, the ethers, the amines, the amides, and the esters, and mixtures thereof.

The primary preferred anti-solvent is methanol, although the anti-solvent for substantially total TPA precipitation from the mother liquor can also be selected from various polar organic solvents including, but not intended to be limited to, methyl ethyl ketone, acetone, C, to C₁₂ alcohols, the carbitols, the esters, the ethers, C₁ to C₁₂ carboxylic acids, water, and mixtures thereof.

The primary preferred selective crystallization solvent for IPA purification is methanol, although the solvent can also be selected from the group, but not limited to, methyl ethyl ketone, acetone, C₁ to C₁₂ alcohols, the carbitols, the esters, ethers, C₁ to C₁₂ carboxylic acids, water, and mixtures thereof.

In order to remove the residual solvent (*e*.*g*., NMP) trapped in the crystals of the final TPA product, the washed TPA crystals are preferably fed to a high temperature soaker where water is used to partially or completely dissolve the TPA crystals. The residual solvent (methanol) trapped in the crystals of the final IPA can be removed by drying to the level of less than 0.25%.

In one embodiment, the method of the present invention comprises the steps of: (a) dissolving the crude TPA in a selective crystallization solvent at a temperature of from 50°C to 250°C to form a solution; (b) crystallizing purified acid from said solution by reducing the temperature and/or pressure thereof, (c) separating said crystallized purified TPA from said solution; (d) redissolving said separated purified TPA in a selective crystallization solvent to form a second solution; (e) crystallizing second stage purified TPA from said second solution by reducing the temperature and pressure sufficient to flash evaporate solvent from said TPA of said second solution but without cooling said solution below 50°C; (f) separating said second stage purified TPA from said second solution; (g) washing said separated second stage purified TPA with water; (h) soaking said washed separated second stage purified TPA with water at a temperature between 150°C and about 300°C; (i) filtering and drying said water soaked second stage purified TPA; (j) adding an anti-solvent to said filtered solution in (c) to cause the precipitation of substantially all the TPA; (k) separating said precipitated TPA from said solution in step (j) and combining said precipitated TPA with said the original crude TPA for processing in step (a); (l) evaporating the solvents from said filtered TPA-free solution in step (k) to cause the crystallization of IPA at a temperature from 5°C and 100°C; (m)
separating said crystallized crude IPA from said solution in step (l); (n) redissolving crude IPA in a selective crystallization solvent at a temperature from 50 to 250°C to form a second solution; (o) crystallizing purified IPA from said second solution in step (n) by reducing the temperature and pressure sufficient to flash evaporate solvent from said IPA of said second solution but without cooling said solution below 50°C; and (p) separating and drying said second stage purified IPA from said second solution.

In this embodiment, the dispersion contains up to 20% isophthalic acid (IPA), and minor amounts of 4-carboxyaldehyde (4-CBA), 3-carboxyaldehyde (3-CBA) and impurities selected from unreacted starting materials, solvents, products of side reactions and/or other undesired materials. The selective crystallization solvent for TPA purification is selected from the group consisting of N-methyl pyrrolidone, (NMP), N,N-dimethyl acetamide, N,N-dimethyl formamide, N-formyl piperidine, N-alkyl-2-pyrrolidone (such as N-ethyl pyrrolidone), N-mercaptoalkyl-2-pyrrolidone (such as N-mercaptoethyl-2-pyrrolidone), N-alkyl-2-thiopyrrolidone (such as N-methyl-2-thiopyrrolidone), N-hydroxyalkyl-2-pyrrolidone (such as N-bydroxyethyl-2-pyrrolidone), the morpholines (such as morpholine, and N-formyl morpholine), the carbitols, C₁ to C₁₂ alcohols, the ethers, the amines, the amides, and the esters, and mixtures thereof The selective crystallization solvent for TPA purification in this embodiment is N-methyl pyrrolidone or N,N-dimethyl acetamide or N-methyl pyrrolidone. The anti-solvent for TPA precipitation from TPA/IPA solution is selected from the group consisting of methanol, water, methyl ethyl ketone, acetone, C₁ to C₁₂ alcohols, the carbitols, the esters, the ethers, C₁ to C₁₂ carboxylic acids, water, and mixtures thereof The selective crystallisation solvent for re-crystallization of IPA is selected from the group of methanol, water, methyl ethyl ketone, acetone, C₁ to C₁₂ alcohols, the carbitols, the esters, ethers, C₁ to C₁₂ carboxylic acids, water, and mixtures thereof The anti-solvent is preferably at the antisolvent/solution ratio of 0.1 to 10, and more preferably at a ratio of between 0.5 to 3, to cause the precipitation of TPA.

In another embodiment, the dispersion contains up to 20% isophthalic acid (IPA) and minor amounts of 4-carboxyaldehyde (4-CBA), 3-carboxyaldehyde (3-CBA), and impurities selected from unreacted starting materials, solvents, products of side reactions and/or other undesired materials. The selective crystallization solvent for TPA purification is selected from the group consisting of N-methyl pyrrolidone (NMP), N,N-dimethyl acetamide, N,N-dimethyl formamide, N-formyl piperidine, N-alkyl-2-pyrrolidone (such as N-ethyl pyrrolidone), N-mercaptoalkyl-2-pyrrolidone (such as N-mercaptoethyl-2-pyrrolidone), N-alkyl-2-thiopyrrolidone (such as N-methyl-2-thiopyrrolidone), N-hydroxyalkyl-2-pyrrolidone (such as N-hydroxyethyl-2-pyrrolidone), the morpholines (such as morpholine, and N-formyl morpholine), the carbitols, C₁ to C₁₂ alcohols, the ethers, the amines, the amides, and the esters, and mixtures thereof. The selective crystallization solvent for TPA purification is N-methyl pyrrolidone or N,N-dimethyl acetamide.

The following examples illustrate the effectiveness of the selective crystallization solvent in separating TPA and IPA, which is the principle and feature of this invention:

### Example 1

This example describes the experimental data on the solubility of TPA as well as IPA in NMP as the selective crystallization solvent at three different temperatures under atmospheric pressure. The experiments were conducted in a laboratory flask which was immersed in a constant temperature bath kept at a pre-determined temperature. The liquid phase temperature in the flask was measured by a thermometer. For high temperature measurement, a total reflux condenser was used to recover the solvent losses due to evaporation. During an experimental run, a small incremental quantity of solids was added to the constantly stirred solvent in the flask until no more solids were dissolved and the solution was then considered saturated with the solids at that temperature. The solubility was calculated based on the weight of solvent and the total weight of solids added. Table 1 summarizes the solubility of TPA and IPA in NMP at 15, 40, 70, and 160°C.

**Table 1**

| **Solid** | **Solvent** | **Solubility (gm of solids/100 gm of solvent)** | | | |
|---|---|---|---|---|---|
| | | **15°C** | **40°C** | **70°C** | **160°C** |
| TPA | NMP | 2.8 | 8.0 | 14.0 | 23.0 |
| IPA | NMP | 10.7 | 22.2 | 46.0 | 62.0 |
| 4-CBA | NMP | 18.9** | 27.4 | 66.0 | 125.0* |

| | | | | | |
|---|---|---|---|---|---|
| * At 110°C | | | | | |
| ** At 23°C | | | | | |

Based on the solubility data shown in Table 1, it is illustrated that TPA can be purified from the mixture of TPA, IPA, and 4-CBA (3-CBA) by crystallization, since both IPA and CBAs tend to remain in the mother liquor due to their higher solubility. The TPA crystals yielded from the mother liquor should have a substantially higher portion of TPA relative to other components than those contained in the mother liquor

### Example 2

A solid mixture containing approximately 95 wt% TPA and 5 wt% IPA was added to NMP according to the solubility of TPA in NMP at 160°C. The mixture was then transferred to a cooling crystallizer equipped with a specially designed mixer in order to minimize the crystal breakage, a heating jacket, and a vapor condenser. The crystallizer was slowly heated up to 160°C and maintained at this temperature for one hour to ensure all the solids were dissolved. The crystallizer was then cooled down to 45°C in 90 minutes to allow the TPA crystals to grow. The crystallizer content was transferred to a jacketed filter and filtered quickly while maintaining the temperature at 40 to 45°C. An appropriate amount of warm solvent (at 50 to 70°C) was used to wash the cake. In some cases, a hot water wash of the cake at 95°C was carried out after a warm solvent wash The washed cake was dried and analyzed by gas chromatography to determine the product composition. Table 2 sets forth a summary of the results.

**Table 2**

| Run Number | Filter Temp (°C) | IPA in Feed | IPA in product | Rinse Condition |
|---|---|---|---|---|
| 1A | 41 | 4.94 wt% | 0.21 wt% | 3 times solvent used at 50°C |
| 2A | 41 | 5.02 wt% | 0.16 wt% | 3 times solvent used at 70°C |
| 3A | 41 | 5.00 wt% | 0.18 wt% | 3 times solvent used at 53°C |
| 1B | 41 | 4.94 wt% | 0.15 wt% | same as 1 A plus 10 times water at 95°C |
| 2B | 41 | 5.02 wt% | 0.13 wt% | same as 2A plus 10 times water |
| 3B | 41 | 5.00 wt% | 0.15 wt% | same as 3B plus 10 times water at 95°C |

The data in Table 2 above demonstrates that the IPA content in TPA was surprisingly reduced (23 to 39 times) depending upon the rinse condition. When the TPA cake from crystallization was rinsed with solvent at 70°C followed by a water rinse at 95°C (Run 2B), the IPA content was indeed reduced 39 times by a single-stage crystallization. Following the same procedure. IPA content in TPA mixture can be reduced from 5 wt% to 33 parts per million by weight (ppmw) through a two-stage crystallization.

### Example 3

This example gives the experimental data on the solubility of TPA as well as IPA in methanol as the selective crystallization at various temperatures under atmospheric pressure. The experimental apparatus and procedures are the same as those set forth for Example 1 above, with the exception that the vapor pressure is greater than atmospheric. The solubility was calculated based on the weight of solvent and the total weight of solids added. Table 3 summarizes the solubility of PTA and IPA in methanol at various temperatures.

**Table 3**

| **Temperature (°C)** | **Solubility (gm of solid/100gm of methanol)** | |
|---|---|---|
| | **TPA** | **IPA** |
| 10 | | 0.03 |
| 25 | 0.09 | 1.82 |
| 50 | 0.47 | 4.00 |
| 160 | | 2.90 |
| 161 | | 15.00 |

It is observed from Table 3 that the solubility of IPA in methanol is roughly 8 to 20 times higher than that of TPA at the temperature ranging from 25 to 50°C. The solubility of TPA in methanol becomes significant only at higher temperatures, such as 160 to 200°C under pressure.

### Example 4

From Example 3, it was found that the solubility of IPA in methanol is substantially higher than that of TPA at room temperature (25°C to 50°C). Accordingly, experiments were conducted to determine whether the precipitation of TPA from the solution of TPA. IPA and a minor amount of 4-CBA could be effectuated by adding the proper amount of methanol to the solution. This solution can be the mother liquor from the TPA crystallizer after the TPA crystals are removed with a filter. The mother liquor may have the following composition: 100 grams of NMP, 20 grams of TPA, 10 grams of IPA, and a minor amount of 4-CBA (and 3-CBA)

This example shows that adding methanol to the mother liquor can cause essentially total precipitation of TPA but only minor precipitation of IPA. To a mixture of 100 grams of NMP, 4 grams of TPA, and 1.5 grams of IPA, approximately 210 grams of methanol was added to the room temperature mixture. The total mixture was stirred for about 90 minutes to allow the solids to crystallize and precipitate from the mixture The crystals were filtered, washed and dried for analysis. It was found that 47.5% of TPA in the mother liquor was recovered. and the crystals contained roughly 99.0 wt% TPA and 1.0 wt% IPA.

To increase the recovery of TPA. the mother liquor was concentrated by removing a part of NMP and the mixture contained 100 grams of NMP, 20 grams of TPA and 10 grams of IPA. Approximately 260 grams of methanol was added to the mixture to cause TPA to crystallize from the mixture at room temperature Upon the addition of methanol, the mixture was stirred for 90 minutes before filtering the TPA crystals from the slurry. It was found up to 97.5% TPA was recovered from the mother liquor. and the TPA crystals contained 97.3 wt% TPA and 2.7 wt% IPA. The data indicates that 100% TPA recovery can be achieved by removing more NMP from the mother liquor (higher concentration), or by adding more methanol to the mother liquor, or the combination of both.

In a typical mother liquor, the 4-CBA content should be around 0.01 grams per 100 grams of NMP (0.01%). Since the amount of 4-CBA in the mother liquor is very small and the solubility of 4-CBA in NMP is very high around room temperature (shown in Table 1), the addition of methanol should not cause the precipitation of 4-CBA from the mother liquor.

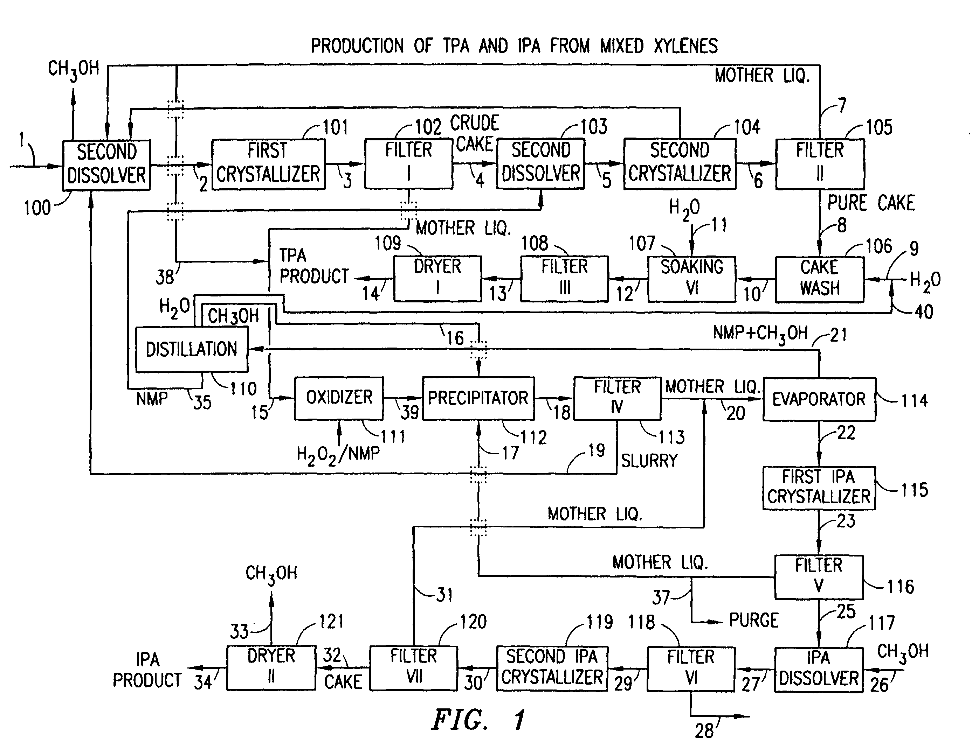

After 1.00% of TPA from the mother liquor is recovered and recycled by adding methanol, the TPA-free mother liquor can be further processed to recover IPA. Details of the process scheme is presented in Figure 1 and is described later in the next section.

### DESCRIPTION OF THE DRAWINGS

Figure 1 is a diagrammatic flow chart for a plant for practicing a preferred embodiment of the invention for producing both TPA and IPA: and
Figure 2 is a diagrammatic flow chart for a plant for practicing another preferred embodiment of the invention for producing TPA only.

### DESCRIPTION OF THE PREFERRED EMBODIMENTS

One of the preferred embodiments of this invention for producing both purified TPA and IPA is presented in Figure 1.

Now referring to FIGURE 1, crude TPA from the oxidation section of a reactor (not shown) containing approximately 95% TPA, 5% IPA, and minor amounts of other impurities (4-CBA, 3-CBA. p-toluic acid, m-toluic acid, etc.), is fed to the First Dissolver 100 through Line 1 to mix with the mother liquor from Filter II 105 (through Line 7) and the slurry from Filter IV 113 (through Line 19). The temperature in the First Dissolver 100 is maintained at 160 to 180°C to completely dissolve the solids and to evaporate substantially all methanol carried over from Line 19.

The saturated solution from the First Dissolver 100 is then fed continuously to First Cooling Crystallizer 101 through Line 2 to generate TPA salt crystals at 30 to 50°C. The slurry containing TPA salt crystals exit First Cooling Crystallizer 101 through Line 3 to Filter I 102 where the crude crystal cake is removed and fed to Second Dissolver 103 through Line 4. In Second Dissolver 103, the cake is re-dissolved in clean NMP recycled through Line 35 from the solvent recovery system. Again, the temperature in Second Dissolver 103 is kept at 160 to 180°C to completely dissolve the TPA salt crystals. The saturated solution from Second Dissolver 103 is continuously fed through Line 5 to Second Flash Crystallizer 104 where the temperature is maintained at a minimum of 60°C to prevent the formation of TPA salt crystals. The degree of temperature reduction in the crystallizer is controlled by the amount of NMP flashed from the crystallizer through pressure reduction The flashed NMP is recycled to the First Dissolver 100 through Line 36.

The slurry from Second Crystallizer 104 is fed to Filter II 105 through Line 6 where the purified TPA solid cake is recovered and sent to Cake Wash 106, while the mother liquor is recycled to First Dissolver 100 through Line 7. In Cake Wash unit 106, the bulk residual NMP in the cake is removed by counter-current washing with water and the washed cake is fed through Line 10 to a Soaker 107 to remove the final trace of NMP in the TPA solids by soaking with water at temperatures between 160 to 280°C The NMP-free cake is filtered in Filter III 108 and dried in Dryer I 109 to yield the final TPA product

The mother liquor from Filter I 102 is transferred through Line 15 to Precipitator 112. In doing so, it passes through Oxidizer 111, which is useful in the practice of a related invention disclosed and claimed in co-pending U.S. Application Serial No. 09/098, 060. entitled "Method to Reduce Carboxybenzaldehyde Isomers in Terephthalic Acid or Isophthalic Acid." owned by the assignee of the present application. whose disclosure is incorporated herein by reference for all purposes. Methanol is added to the precipitator through Line 16 to cause the complete precipitation (or crystallization) of TPA and a small amount of precipitation of IPA from the mother liquor The slurry from Precipitator 112 is fed to Filter IV 113 through Line 18 to remove the major portion of the mother liquor from the slurry before it is recycled to First Dissolver 100 through Line 19.

The mother liquor from Filter IV 113 is sent to Evaporator 114 to remove NMP and methanol by evaporation through heat as well as vacuum. so that the concentrated mother liquor becomes a saturated solution of IPA, which is fed to First IPA Crystallizer 115 to crystallize IPA at a temperature between 30 to 50°C by cooling or flashing. The vaporized NMP and methanol from Evaporator 114 is fed to Distillation Column 110 to yield NMP from the bottom and methanol from the top of the column. The methanol stream is recycled to Precipitator 112 through Line 16, while the NMP stream is fed to Second Dissolver 103 through Line 35. The slurry from First IPA Crystallizer 115 is transferred to Filter V 116 to produce crude IPA cake and the mother liquor. The mother liquor is sent to Precipitator 112 through Line 17, but a portion of Stream 17 is purged through Line 37 to prevent the accumulation of the impurities and color bodies.

The cake from Filter V 116 is then transferred through Line 25 to IPA Dissolver 117 where the crude IPA cake is dissolved by methanol at a suitable temperature and pressure The saturated IPA solution is filtered in Filter VI 118 to remove the trace insolubles for purging through Line 28. The solid-free solution is fed through Line 29 to Second IPA Crystallizer 119 to yield IPA crystals by flashing methanol from the crystallizer through pressure reduction. The slurry from Second IPA Crystallizer 119 is transferred through Line 30 to Filter VII 120 to recover and wash the purified IPA crystals for final drying in Dryer II 121 to yield the final IPA product, while the mother liquor from Filter VII 120 is recycled the Evaporator 114 through Line 31.

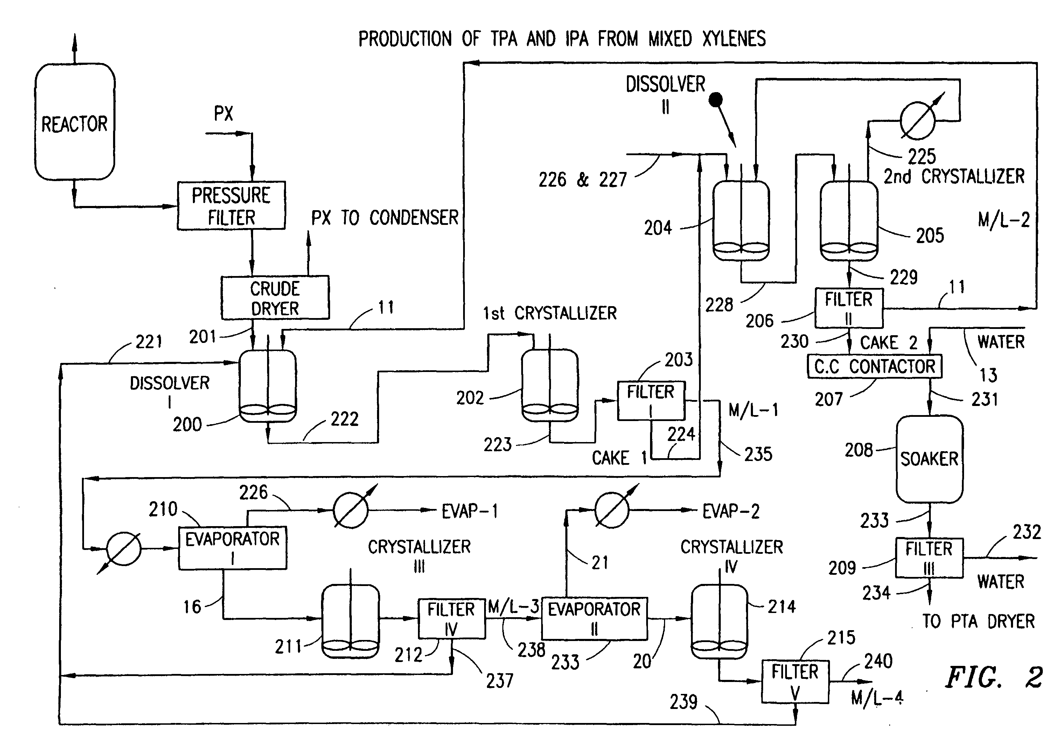

Another preferred embodiment of this invention for producing purified TPA only is illustrated in Figure 2. The crude TPA (containing roughly 90 to 99% TPA and 1 to 10% IPA) is fed to Dissolver I 200 through Line 201 to mix with the mother liquor M/L-2 from Filter II 206 (recycled through Line 220) and the recycled cake from Filter V 215 (recycled through Line 221). The temperature in the dissolver is maintained at 140 to 200°C to dissolve substantially all the solids. The saturated solution is then fed through Line 222 to the First Crystallizer 202 where the temperature is reduced to 30 to 60°C by cooling or solvent evaporation (with pressure reduction) to allow the TPA crystals to grow

The slurry from First Crystallizer 202 is transferred continuously or batchwise through Line 223 to Filter I 203 to recover the solid cake. Washing the lean or saturated NMP is required at Filter I 203 to displace the mother liquor from the cake before it is transferred through Line 224 to Dissolver II 204, where the cake is mixed with the flashed NMP from Line 225 and evaporated NMP from Lines 226 and 227. Again, the temperature in Dissolver II 204 is maintained at 140 to 200°C to dissolve substantially all the solids. The saturated solution is fed through Line 228 to Second Crystallizer 205 where the temperature is reduced to 30 to 60°C by cooling or solvent evaporation (with pressure reduction) to allow the purified TPA crystals to grow.

Again, the slurry from Second Crystallizer 205 is fed through Line 229 to Filter II 206 to recover the cake, which is then transferred through Line 230 to a Countercurrent Contactor 207 to be washed with water to remove the bulk of free NMP from the cake. The water-washed solids are sent through Line 231 to Soaker 208 to remove the trace amount of trapped NMP from the purified TPA solids by partial or total dissolving of the solids in Soaker 208 at a temperature of 150 to 280°C. The NMP-free solids are sent through Line 233 to Filter III 209 where the water is removed through Line 232 and the TPA cake is sent through Line 234 to be dried in a dryer to yield the final purified TPA product.

The mother liquor M/L-1 from Filter I 203 is sent through Line 235 to Evaporator 1 210 to remove a substantial amount of NMP. The concentrated solution is transferred through Line 236 to Crystallizer III 211 to cause low-purity TPA crystals to grow. The crystals are then recovered from Filter IV 212 and recycled to Dissolver I 200 through Line 237. The mother liquor M/L-3 from Filter IV 212 is transferred via Line 238 to Evaporator II 213, then Crystallizer IV 214 and Filter V 215 to recover the residual low-purity TPA for recycling to Dissolver 1200 via Line 239 The final mother liquor M/L-4 from Filter V 215 containing mainly IPA, NMP and a minor amount of TPA passes through Line 240 and is to be treated for further NMP recovery by mixing with water before disposal.

## Claims

1. A process for separating and purifying crude terephthalic acid (TPA) and isophthalic acid (IPA) from a liquid dispersion consisting of TPA, IPA and other impurities produced from the oxidation of mixed xylenes comprising:
(a) dissolving the crude TPA in a selective crystallization solvent at a temperature of from 50°C to 250°C to form a solution;
(b) crystallizing purified acid from said solution by reducing the temperature and/or pressure thereof, and separating said crystallized purified TPA from said solution;
(c) adding an anti-solvent to the said solution from which said crystallized purified TPA is separated to cause the precipitation of substantially all the TPA remaining in said solution;
(d) separating said precipitated TPA;
(e) evaporating the solvents from the solution to cause crystallization of IPA, and purifying and recovering purified IPA thereby.

2. The process of claim 1 further comprising prior to step (c);
(i) redissolving said separated purified TPA in a selective crystallization solvent to form a second solution;
(ii) crystallizing second stage purified TPA from said second solution by reducing the temperature and pressure sufficient to flash evaporate solvent from said TPA of said second solution but without cooling said solution below 50°C;
(iii) separating said second stage purified TPA from said solution;
(iv) washing said separated second stage purified TPA with water;
(v) soaking said washed separated second stage purified TPA with water at a temperature between 150°C and 300°C;
(vi) filtering and drying said water soaked second stage purified TPA, and wherein step (d) of claim 1 further comprises;
(vii) separating said precipitated TPA from said solution in (c) and combining said precipitated TPA with said original crude TPA for processing in (a); and step (e) of claim 1 further comprises;
(viii) evaporating the solvents from said filtered TPA-free solution in (vii) to cause the crystallization of IPA at a temperature from 5°C and 100°C;
(ix) separating said crystallized crude IPA from said solution in (viii),
(x) redissolving crude IPA in a selective crystallization solvent at a temperature from 50°C to 250°C to form a second solution;
(xi) crystallizing purified IPA from said second solution in (x) by reducing the temperature and pressure sufficient to flash evaporate solvent from said IPA of said second solution but without cooling said solution below 50°C; and
(xii) separating and drying said second stage purified IPA from said second solution.

3. The process of claims 1 or 2 in which said dispersion contains up to 20% isophthalic acid (IPA), and minor amounts of 4-carboxyaldehyde (4-CBA), 3-carboxyaldehyde (3-CBA) and impurities selected from unreacted starting. materials, solvents, products of side reactions and/or other undesired materials.

4. The process of claim 1 or 2 wherein said selective crystallization solvent for TPA purification is selected from the group consisting of N-methyl pyrrolidone (NMP), N,N-dimethyl acetamide, N,N-dimethyl formamide. N-formyl piperidine, N-alkyl-2-pyrrolidone, N-mercaptoalkyl-2-pyrrolidone, N-alkyl-2-thiopyrrolidone, N-hydroxyalkyl-2-pyrrolidone, the morpholines, the carbitols, C₁ to C₁₂ alcohols, the ethers, the amines, the amides, and the esters, and mixtures thereof.

5. The process of claim 4 wherein said selective crystallization solvent for TPA purification is N-methyl pyrrolidone or N,N-dimethyl acetamide.

6. The process of claim 5 wherein said selective crystallized solvent for TPA purification is N-methyl pyrrolidone.

7. The process of claim 1 or 2 wherein said anti-solvent for TPA precipitation from TPA/IPA solution is selected from the group consisting of methanol, water, methyl ethyl ketone, acetone, C₁ to C₁₂ alcohols, the carbitols, the esters, the ethers, C₁ to C₁₂ carboxylic acids, water and mixtures thereof.

8. The process of claim 7 wherein said anti-solvent for TPA precipitation from TPA/IPA solution is methanol or water.

9. The process of claim 8 wherein said anti-solvent for TPA precipitation from TPA/IPA solution is methanol.

10. The process of claim 1 or 2 wherein said selective crystallization solvent for recrystallization of IPA is selected from the group of methanol, water, methyl ethyl ketone, acetone, C₁ to C₁₂ alcohols, the carbitols, the esters, ethers, C₁ to C₁₂ carboxylic acids, water and mixtures thereof.

11. The process of claim 10 wherein said selective crystallization solvent for recrystallizing purified IPA is methanol or water.

12. The process of claim 1 or 2 wherein the said anti-solvent is at the anti-solventlsolution ratio of 0.1 to 10 to cause the precipitation of TPA.

13. The process of claim 12 wherein the said anti-solvent/solution ratio is preferably in the range of 0.5 to 3.

14. The process of claim 1 and further comprising dissolving and crystallizing said purified IPA to obtain IPA of desired purity.

15. The process of claim 1 wherein the method further comprises repeating said dissolution and crystallizing of said TPA, if necessary, to obtain a purified TPA of desired purity prior to adding the anti-solvent.

## Patentansprüche

1. Verfahren zum Trennen und Reinigen von roher Terephthalsäure (TPA) und Isophthalsäure (IPA) aus einer flüssigen Dispersion, bestehend aus TPA, IPA und anderen Verunreinigungen, die bei der Oxidation von einem XylolGemisch erhalten werden, umfassend:
(a) Lösen von roher TPA in einem selektiven Kristallisationslösungsmittel bei einer Temperatur von 50°C bis 250°C, so dass man eine Lösung erhält;
(b) Kristallisieren der gereinigten Säure aus der Lösung durch Senken ihrer Temperatur und/oder ihres Drucks, und Abtrennen der kristallisierten gereinigten TPA aus der Lösung;
(c) Zugeben eines Antilösungsmittels zu der Lösung, aus der die kristallisierte gereinigte TPA abgetrennt wird, so dass im Wesentlichen die gesamte in der Lösung verbliebene TPA gefällt wird;
(d) Abtrennen der gefällten TPA;
(e) Verdampfen der Lösungsmittel aus der Lösung, so dass die IPA kristallisiert, und dadurch Reinigen und Gewinnen von gereinigter IPA.

2. Verfahren nach Anspruch 1, zudem umfassend vor Schritt (c);
(i) Wiederauflösen der getrennten gereinigten TPA in einem selektiven Kristallisations-Lösungsmittel, so dass man eine zweite Lösung erhält;
(ii) Kristallisieren der in der zweiten Stufe gereinigten TPA aus der zweiten Lösung, indem die Temperatur und der Druck so stark gesenkt werden, dass das Lösungsmittel aus der TPA der zweiten Lösung flash-verdampft, jedoch ohne Kühlen der Lösung unter 50°C;
(iii) Trennen der in der zweiten Stufe gereinigten TPA aus der Lösung;
(iv) Waschen der in der zweiten Stufe gereinigten TPA mit Wasser;
(v) Wässern der gewaschenen, getrennten und in der zweiten Stufe gereinigten TPA mit Wasser bei einer Temperatur zwischen 150°C und 300°C;
(vi) Filtrieren und Trocknen der gewässerten, in der zweiten Stufe gereinigten TPA,
wobei:
Schritt (d) von Anspruch 1 zudem umfasst:
(vii) Trennen der gefällten TPA aus der Lösung in (c) und Vereinigen der gefällten TPA mit der ursprünglichen rohen TPA zur Verarbeitung in (a);
und Schritt (e) von Anspruch 1 zudem umfasst:
(viii) Eindampfen der Lösungsmittel aus der filtrierten TPA-freien Lösung in (vii), so dass die IPA bei einer Temperatur von 5°C und 100°C kristallisiert;
(ix) Trennen der kristallisierten rohen IPA aus der Lösung in (viii);
(x) Wiederauflösen der rohen IPA in einem selektiven Kristallisations-Lösungsmittel bei einer Temperatur von 50°C bis 250°C, so dass man eine zweite Lösung erhält;
(xi) Kristallisieren der gereinigten IPA aus der zweiten Lösung in (x), indem die Temperatur und der Druck so stark gesenkt werden, dass das Lösungsmittel aus der IPA der zweiten Lösung flash-verdampft, jedoch ohne Kühlen der Lösung unter 50°C; und
(xii) Trennen und Trocknen der in der zweiten Stufe gereinigten IPA aus der zweiten Lösung.

3. Verfahren nach den Ansprüchen 1 oder 2, wobei die Dispersion bis zu 20% Isophthalsäure (IPA) und geringe Mengen 4-Carboxyaldehyd (4-CBA), 3-Carboxyaldehyd (3-CBA) und Verunreinigungen enthält, die ausgewählt sind aus nicht umgesetzten Ausgangsmaterialien, Lösungsmitteln, Produkten von Nebenreaktionen und/oder ungewünschten Materialien.

4. Verfahren nach Anspruch 1 oder 2, wobei das selektive Kristallisations-Lösungsmittel zur TPA-Reinigung ausgewählt ist aus der aus N-Methylpyrrolidon (NMP), N,N-Dimethylacetamid, N,N-Dimethylformamid, N-Formylpiperidin, N-Alkyl-2-pyrrolidon, N-Mercaptoalkyl-2-pyrrolidon, N-Alkyl-2-thiopyrrolidon, N-Hydroxyalkyl-2-pyrrolidon, Morpholinen, Carbitolen, C₁-C₁₂-Alkoholen, Ethern, Aminen, Amiden und Estern sowie deren Gemischen bestehenden Gruppe.

5. Verfahren nach Anspruch 4, wobei das selektive Kristallisations-Lösungsmittel zur TPA-Reinigung N-Methylpyrrolidon oder N,N-Dimethylacetamid ist.

6. Verfahren nach Anspruch 5, wobei das selektive Kristallisations-Lösungsmittel zur TPA-Reinigung N-Methylpyrrolidon ist.

7. Verfahren nach Anspruch 1 oder 2, wobei das Antilösungsmittel zur TPA-Fällung aus der TPA/IPA-Lösung ausgewählt ist aus der aus Methanol, Wasser, Methylethylketon, Aceton, C₁-C₁₂-Alkoholen, Carbitolen, Estern, Ethern, C₁-C₁₂-Carbonsäuren, Wasser und deren Gemischen bestehenden Gruppe.

8. Verfahren nach Anspruch 7, wobei das Antilösungsmittel zur TPA-Fällung aus der TPA/IPA-Lösung Methanol oder Wasser ist.

9. Verfahren nach Anspruch 8, wobei das Antilösungsmittel zur TPA-Fällung aus der TPA/IPA-Lösung Methanol ist.

10. Verfahren nach Anspruch 1 oder 2, wobei das selektive Kristallisations-Lösungsmittel zur Umkristallisation von IPA ausgewählt ist aus der aus Methanol, Wasser, Methylethylketon, Aceton, C₁-C₁₂-Alkoholen, Carbitolen, Estern, Ethern, C₁-C₁₂-Carbonsäuren, Wasser und deren Gemischen bestehenden Gruppe.

11. Verfahren nach Anspruch 10, wobei das selektive Kristallisations-Lösungsmittel zur Umkristallisation von gereinigter IPA Methanol, Methanol oder Wasser ist.

12. Verfahren nach Anspruch 1 oder 2, wobei das Antilösungsmittel in einem Verhältnis von Antilösungsmittel zu Lösung von 0,1 bis 10 vorliegt, damit TPA gefällt wird.

13. Verfahren nach Anspruch 12, wobei das Verhältnis von Antilösungsmittel zu Lösung vorzugsweise in dem Bereich von 0,5 bis 3 ist.

14. Verfahren nach Anspruch 1, zudem umfassend das Lösen und Kristallisieren der gereinigten IPA, so dass eine IPA der gereinigten Reinheit erhalten wird.

15. Verfahren nach Anspruch 1, wobei das Verfahren zudem nötigenfalls das Wiederholen von Auflösen und Kristallisieren der TPA umfasst, so dass man eine gereinigte TPA der gewünschten Reinheit vor der Zugabe des Antilösungsmittels erhält.

## Revendications

1. Processus pour séparer et purifier l'acide téréphtalique (TPA) et l'acide isophtalique (IPA) bruts à partir d'une dispersion liquide constituée de TPA, d'IPA et d'autres impuretés produites par l'oxydation de xylènes mixtes comprenant:
(a) la dissolution du TPA brut dans un solvant de cristallisation sélectif à une température de 50 à 250°C pour former une solution ;
(b) la cristallisation de l'acide purifié à partir de ladite solution en réduisant sa température et/ou sa pression, et la séparation dudit TPA purifié cristallisé de ladite solution ;
(c) l'addition d'un anti-solvant à ladite solution à partir de laquelle ledit TPA cristallisé purifié est séparé pour entraîner la précipitation d'essentiellement tout le TPA restant dans ladite solution;
(d) la séparation dudit TPA précipité ;
(e) l'évaporation des solvants de la solution pour entraîner la cristallisation de l'IPA, et la purification et la récupération de l'IPA ainsi purifié.

2. Processus selon la revendication 1 comprenant en outre avant l'étape (c) :
(i) la redissolution dudit TPA séparé purifié dans un solvant de cristallisation sélectif pour former une deuxième solution ;
(ii) la cristallisation du TPA purifié au deuxième stade de ladite deuxième solution en réduisant la température et la pression suffisamment pour évaporer instantanément le solvant dudit TPA de ladite deuxième solution mais sans refroidir ladite solution au-dessous de 50°C ;
(iii) la séparation dudit TPA purifié au deuxième stade de ladite solution ;
(iv) le lavage dudit TPA purifié au deuxième stade séparé avec de l'eau;
(v) le trempage dudit TPA purifié au deuxième stade séparé lavé avec de l'eau à une température entre 150 et 300°C ;
(vi) la filtration et le séchage dudit TPA purifié au deuxième stade trempé dans l'eau et dans lequel l'étape (d) de la revendication 1 comprend en outre :
(vii) la séparation dudit TPA précipité de ladite solution en (c) et la combinaison dudit TPA précipité avec ledit TPA brut original pour le traitement en (a) ;
et l'étape (e) selon la revendication 1 comprend en outre:
(viii) l'évaporation des solvants de ladite solution sans TPA filtrée en (vii) pour entraîner la cristallisation de l'IPA à une température de 5 et 100°C;
(ix) la séparation dudit IPA brut cristallisé de ladite solution en (viii),
(x) la redissolution de l'IPA brut dans un solvant de cristallisation sélectif à une température de 50 à 250°C pour former une deuxième solution ;
(xi) la cristallisation de l'IPA purifié de ladite deuxième solution en (x) en réduisant la température et la pression suffisamment pour évaporer instantanément le solvant dudit IPA de ladite deuxième solution mais sans refroidir ladite solution au-dessous de 50°C ; et
(xii) la séparation et le séchage dudit IPA purifié au deuxième stade de ladite deuxième solution.

3. Processus selon les revendications 1 ou 2, dans lequel ladite dispersion contient jusqu'à 20 % d'acide isophtalique (IPA), et des quantités mineures de 4-carboxyaldéhyde (4-CBA), de 3-carboxyafdéhyde (3-CBA) et des impuretés choisies parmi les matériaux de départ n'ayant pas réagi, les solvants, les produits de réactions secondaires et/ou d'autres matériaux non souhaités.

4. Processus selon la revendication 1 ou 2 dans lequel ledit solvant de cristallisation sélectif pour la purification du TPA est choisi dans le groupe constitué par la N-méthyl pyrrolidone (NMP), le N,N-diméthyl acétamide, le N,N-diméthyl formamide, la N-formyl pipéridine, la N-alkyl-2-pyrrolidone, la N-mercaptoalkyl-2-pyrrolidone, la N-alkyl-2-thiopyrrolidone, la N-hydroxyalkyl-2-pyrrolidone, les morpholines, les carbitols, les alcools en C₁ à C₁₂, les éthers, les amines, les amides, et les esters, et leurs mélanges.

5. Processus selon la revendication 4, dans lequel ledit solvant de cristallisation sélectif pour la purification du TPA est la N-méthyl pyrrolidone ou le N,N-diméthyl acétamide.

6. Processus selon la revendication 5, dans lequel ledit solvant cristallisé sélectif pour la purification du TPA est la N-méthyl pyrrolidone.

7. Processus selon la revendication 1 ou 2, dans lequel ledit anti-solvant pour la précipitation du TPA à partir de la solution de TPA/IPA est choisi dans le groupe constitué par le méthanol, l'eau, la méthyléthylcétone, l'acétone, les alcools en C₁ à C₁₂, les carbitols, les esters, les éthers, les acides carboxyliques en C₁ à C₁₂, l'eau et leurs mélanges.

8. Processus selon la revendication 7, dans lequel ledit anti-solvant pour la précipitation du TPA à partir de la solution de TPA/IPA est le méthanol ou l'eau.

9. Processus selon la revendication 8, dans lequel ledit anti-solvant pour la précipitation du TPA à partir de la solution de TPA/IPA est le méthanol.

10. Processus selon la revendication 1 ou 2, dans lequel ledit solvant de cristallisation sélectif pour la recristallisation de l'IPA est choisi dans le groupe du méthanol, de l'eau, de la méthyléthylcétone, de l'acétone, des alcools en C₁ à C₁₂, des carbitols, des esters, des éthers, des acides carboxyliques en C₁ à C₁₂, de l'eau et de leurs mélanges.

11. Processus selon la revendication 10, dans lequel ledit solvant de cristallisation sélectif pour la recristallisation de l'IPA purifié est le méthanol ou l'eau.

12. Processus selon la revendication 1 ou 2, dans lequel ledit anti-solvant est au rapport anti-solvant/solution de 0,1 à 10 pour entraîner la précipitation du TPA.

13. Processus selon la revendication 12, dans lequel ledit rapport anti-solvant/solution est de préférence dans la plage de 0,5 à 3.

14. Processus selon la revendication 1 et comprenant en outre la dissolution et la cristallisation dudit IPA purifié pour obtenir l'IPA de pureté souhaitée.

15. Processus selon la revendication 1, dans lequel le procédé comprend en outre la répétition de ladite dissolution et de la cristallisation dudit TPA, si nécessaire, pour obtenir un TPA purifié de pureté souhaitée avant d'ajouter l'anti-solvant.
